# EUROPEAN PATENT APPLICATION

(11) **EP 1 621 532 A1**
(43) Date of publication of application: **01.02.2006**
(21) Application number: 05014275.1
(22) Date of filing: 30.06.2005
(51) Int. Cl.: C07D 211/22, A61K 31/445

(54) **Fexofenadine base polymorphic forms**

(30) Priority: 30.07.2004 IT mi20041568
(71) Applicant: Dipharma S.p.A., 33036 Mereto di Tomba (Udine) (IT)
(72) Inventor: Ventimiglia, Gianpiero, 20092 Cinisello Balsamo (IT); Allegrini, Pietro, 20097 San Donato Milanese (MI) (IT); Castaldi, Graziano, 28072 Briona (NO) (IT)
(74) Representative: Minoja, Fabrizio

(57) **Abstract**

The invention relates to novel crystalline forms of fexofenadine base, a process for the preparation thereof and the use thereof in therapy.

## Description

### FIELD OF THE INVENTION

The present invention relates to novel crystalline forms of fexofenadine base, a process for the preparation thereof, and the use thereof in therapy.

Fexofenadine, namely (4-[4-[4-(hydroxydiphenylmethyl)-1-piperidinyl]-1-hydroxybutyl]-α,α -dimethylbenzeneacetic acid) 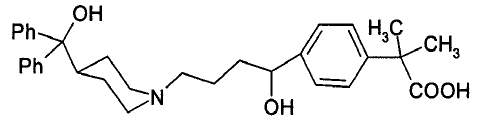 is an antihistaminic, antiallergic and bronchodilator medicament, marketed in the USA under the tradename Allegra®.

A number of processes for the preparation of fexofenadine are known, for example those disclosed in US 4,254,129; US 5,750,703 and EP 1 260 505.

WO 02/066429, US 2003/002184 and WO 95/31437 disclose various polymorphic forms of fexofenadine hydrochloride and WO 03/039482 discloses various polymorphic forms of fexofenadine base.

Different forms of biologically active compounds, such as polymorphs, are known to have different bioavailability, release time and solubility, which allow, for example, dose reduction or prolonged administration intervals. Moreover, the different physical properties that are often associated to different physical forms of medicaments can be advantageously exploited in the manufacture of pharmaceutical formulations.

There is therefore the need to provide novel polymorphic forms of biologically active compounds with advantageous properties.

### SUMMARY OF THE INVENTION

It has now been found that fexofenadine free base can exist, as well as in the known crystalline forms cited above, also in three novel crystalline forms stable at room temperature.

In a first aspect the invention relates to a novel crystalline form of fexofenadine free base, herein referred to as Form α, and a process for its preparation.

In a second aspect the invention relates to a novel crystalline form of fexofenadine free base, herein referred to as Form β, and a process for its preparation.

In a further aspect the invention relates to a novel crystalline form of fexofenadine free base, herein referred to as Form γ, and a process for its preparation.

Moreover, an object of the present invention is the use of the novel crystalline forms of fexofenadine free base as intermediates in a process for the purification of fexofenadine free base or fexofenadine hydrochloride, useful to obtain fexofenadine free base or fexofenadine hydrochloride of quality suitable for the therapeutical use.

Furthermore, the invention provides a pharmaceutical composition containing an excipient and/or carrier and at least an active ingredient consisting of fexofenadine free base Form α, β or γ.

### BRIEF DISCLOSURE OF THE FIGURES

The three novel crystalline forms of Fexofenadine free base were characterized with the XRPD technique (X-ray powder diffraction). The X-ray diffraction spectra (XRPD) were registered with an APD 2000 θ/θ automatic diffractometer for powders and liquids (Ital-Structures), under the following operative conditions: radiation CuKα (1.5418 Å), scanning with a 0.03°C angular step for an acquisition time of 1 sec.
Figure 1. XRPD spectrum of fexofenadine free base Form α.
Figure 2. XRPD spectrum of fexofenadine free base Form β.
Figure 3. XRPD spectrum of fexofenadine free base Form γ.

### DETAILED DISCLOSURE OF THE INVENTION

As used herein, the expressions "fexofenadine free base" and "fexofenadine base" are equivalents and relate to the same product.

As used in the following, the term precipitation (or "precipitate") has the same meaning as crystallization (or "crystal"), and means the obtainment of a solid form.

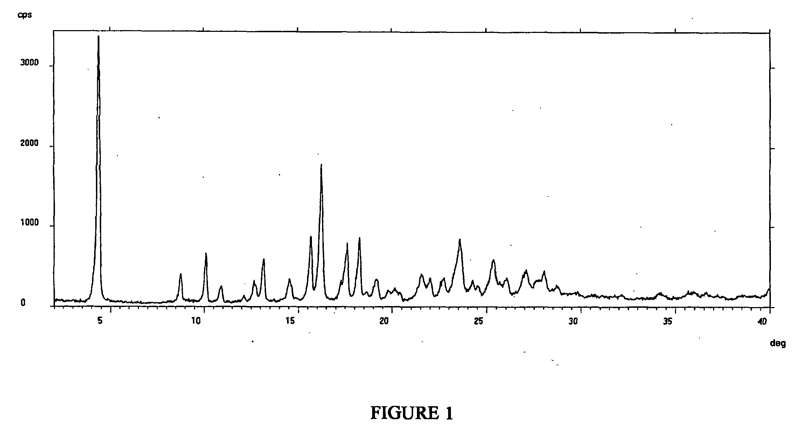

As first object, the present invention relates to a novel crystalline form of fexofenadine base, herein referred to as Form α, having the XRPD spectrum substantially as reported in Figure 1, wherein the most intense diffraction peaks fall at 4.37; 8.75; 10.07; 13.16; 15.65; 16.22 and 23.57 in 2θ.

Said form α is prepared through a process comprising the following steps:
- reaction of a fexofenadine addition salt with a basic organic agent in an organic protic solvent; and
- separation of the precipitate.

A fexofenadine addition salt, used as the starting material for the preparation of the novel Forms α, β and γ of fexofenadine base as reported in the following, can be a salt thereof, either anhydrous or hydrate, with a suitable inorganic acid or organic, for example as known from WO 95/31437. Preferred examples of inorganic acids are hydrochloric, hydrobromic, sulfuric, phosphoric acids. Preferred examples of organic acids are both carboxylic acids, such as acetic, propionic, glycolic, lactic, piruvic acids, or sulfonic acids, such as methanesulfonic, ethanesulfonic and beta-hydroxyethanesulfonic acids. Particularly preferred is fexofenadine hydrochloride salt. As used herein, the expressions "fexofenadine addition salt" and "fexofenadine salt" are equivalents and relate to the same product.

The preparation of said Form α can be carried out starting from a dispersion, for example solution or suspension, of a fexofenadine addition salt in an organic protic solvent. Preferred examples of organic protic solvents, according to the invention, are alcohols, preferably a C₁-C₆ alkanol, such as methanol, ethanol, 1-propanol, 2-propanol or mixtures thereof, particularly methanol. The concentration of the fexofenadine salt in the starting solution or suspension can approximately range from 5 to 50%, preferably from 15 to 30%. The temperature of the resulting solution or suspension can be maintained between the room temperature and the reflux temperature, preferably room temperature. Fexofenadine free base can be directly obtained *in situ* by reacting the corresponding addition salt with a basic organic agent. A basic organic agent is typically ammonia; an amine, e.g. of formula N(R₁R₂R₃), wherein at least one of R₁, R₂ and R₃ is a C₁-C₆ alkyl or aryl group, and the others are independently hydrogen or a C₁-C₆ alkyl or aryl group; or a saturated or unsaturated 5- or 6- membered heterocyclic compound, containing at least one nitrogen atom, for example pyridine, piperidine or morpholine. A straight or branched C₁-C₆ alkyl group is preferably a C₁-C₄ alkyl group, particularly methyl or ethyl. An aryl group is preferably phenyl optionally substituted with one to three substituents independently selected from hydroxy, halogen, cyano and amino. Examples of specific basic organic agents are butylamine, triethylamine, tributylamine and diisopropyl-ethylamine, particularly butylamine, triethylamine and tributylamine. The molar ratio of basic agent to fexofenadine salt approximately ranges from 0.8 to 1.2, preferably from 0.95 to 1.05. The precipitated fexofenadine free base Form α can be recovered with a conventional technique, preferably by filtration, followed by washing with the same solvent or mixtures of solvents as used in the reaction, then dried under vacuum at a temperature depending on the solvent used. This temperature approximately ranges from 0°C to the boiling temperature of the solvent itself, preferably drying is effected at room temperature.

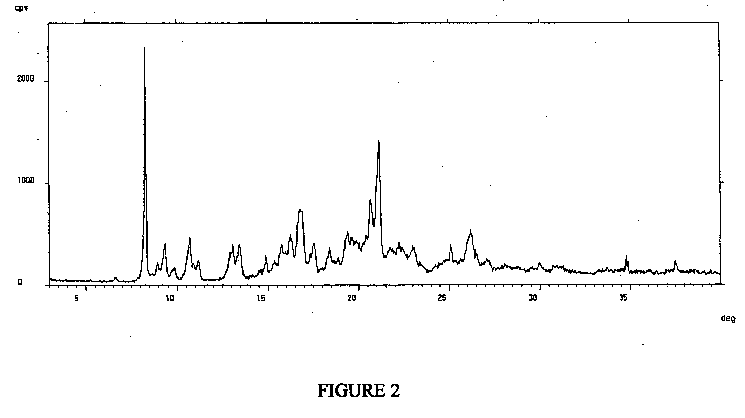

In a second aspect, the invention relates to a novel crystalline form of fexofenadine base, herein referred to as Form β, having the XRPD spectrum substantially as reported in Figure 2, wherein the most intense diffraction peaks fall at 8.28; 10.68; 16.23; 16.80; 19.35 and 21.12 in 2θ.

Said Form β can be prepared by a process comprising the following steps:
- reaction of a fexofenadine addition salt with a basic organic agent in an organic protic solvent;
- acidification of the solution with a carboxylic organic acid to precipitate fexofenadine base Form ß; and
- separation of the precipitate.

The process can be carried out starting from a dispersion, such as a solution or suspension, of a fexofenadine addition salt in an organic protic solvent as defined above, particularly methanol, ethanol, 1-propanol, 2-propanol or mixtures thereof; more preferably methanol. The temperature of the resulting solution or suspension can be kept between the room temperature and the reflux temperature, preferably at room temperature. The fexofenadine free base can be directly obtained *in situ* by reacting the corresponding addition salt with a basic organic agent, as defined above. A particularly preferred example of basic organic agent is triethylamine. The molar ratio of basic agent to fexofenadine addition salt approximately ranges from 1.5 to 3.0, preferably from 1.95 to 2.05. Fexofenadine free base Form β can be precipitated by acidifying again the solution to pH approx. ranging from 4 to 7, preferably from 5 to 6, with a carboxylic organic acid. Said acid can be for example a mono-, bi- or tri- carboxylic acid, such as formic, acetic, oxalic, malonic, maleic, fumaric, tartaric and citric acids. Preferred examples are formic acid and acetic acid, more preferably acetic acid. The resulting solid product can be recovered with conventional techniques, preferably by filtration, followed by washing with the same solvent or mixtures of solvents as used in the reaction, then dried under vacuum at a temperature depending on the solvent used. This temperature approximately ranges from 0°C to the boiling temperature of the solvent, preferably room temperature.

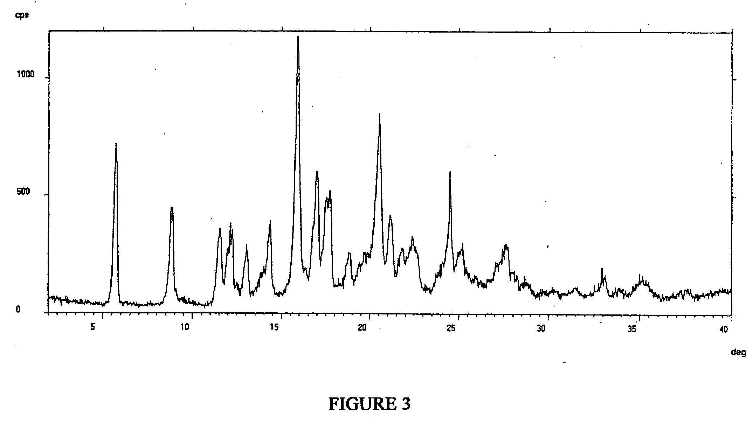

As further object, the present invention relates to a novel crystalline form of fexofenadine base, herein referred to as Form γ, having the XRPD spectrum substantially as reported in Figure 3, wherein the most intense diffraction peaks fall at 5.63; 12.11; 15.83; 16.91; 20.39 and 24.44 in 2θ.

Said Form γ can be prepared by a process comprising the following steps:
- reaction of a fexofenadine addition salt with a basic organic agent in an organic aprotic solvent; and
- separation of the precipitate.

The process can be carried out starting from a dispersion, such as a solution or suspension, of a fexofenadine addition salt in an organic aprotic solvent. Examples of organic aprotic solvents, according to the invention, are acetone, diethyl ketone, methyl ethyl ketone, ethyl acetate, butyl acetate, acetonitrile or mixtures thereof, preferably acetone or acetonitrile or mixtures thereof, particularly acetone or a mixture of acetone and acetonitrile wherein the percentage in volume of acetone in the acetone/acetonitrile mixture approximately ranges from 0.4 to 0.8, preferably approx. from 0.5 to 0.7. The concentration of the fexofenadine salt in the starting solution or suspension can approximately range from 5 to 50%, preferably from about 15 to 30%. The temperature of the resulting solution or suspension can be kept at a temperature ranging between room temperature and the reflux temperature, preferably room temperature. Fexofenadine free base Form γ can be directly obtained *in situ* by reacting the corresponding addition salt with a basic organic agent, as defined above. Preferred examples of basic organic agents are butylamine, tributylamine and triethylamine. The molar ratio of basic agent to fexofenadine salt approximately ranges from 0.8 to 1.2, preferably from 0.95 to 1.05. The precipitated fexofenadine free base Form γ can be recovered with conventional techniques, preferably by filtration, followed by washing with the same solvent or mixtures of solvents used in the reaction and drying under vacuum at a temperature depending on the solvent used. This temperature approximately ranges from 0°C to the boiling temperature of the solvent, preferably 50°C.

The skilled chemist will note that the conditions to obtain the dispersion of a fexofenadine addition salt in a protic or aprotic solvent can be changed without affecting the resulting polymorphic form. Likewise, the crystallization techniques can by slightly changed without affecting the resulting polymorphic form. By way of example, crystallization may be made faster by adding crystals previously formed.

The above recovery process to obtain the novel crystalline forms of the invention allows to purify the final product from any impurities formed during the fexofenadine synthetic process, derived from either parasitic reactions or degradation of the product itself.

Therefore, a further object of the present invention is the use of fexofenadine free base Form α, Form ß or Form γ, as herein defined, in a process for the preparation of fexofenadine free base, or a salt or polymorphic form thereof, having a purity degree suitable for the pharmaceutical or veterinary use.

The novel Forms α, ß and γ of fexofenadine base are useful for the administration of fexofenadine in mammals in need of said treatment. Such forms will be administered alone or as mixtures thereof or mixtures with one or more of the polymorphic forms of fexofenadine base or salt already known, particularly the hydrochloride. The content thereof in the mixtures depends on their physical and biological characteristics and is left to the discretion of those skilled in the art. Such forms can be formulated in a variety of pharmaceutical compositions for the administration to humans or animals, according to known techniques. The dosage of fexofenadine base in the capsules, tablets, sugar-coated pills or other unit forms approximately ranges from 20 to 200 mg; the exact dosage is left to the discretion of the physician.

Therefore the invention also relates to a pharmaceutical composition comprising a suitable carrier and/or excipient and, as active ingredient, at least one of fexofenadine base Form α, Form β or Form γ, or mixtures thereof or mixtures of at least one of them with one or more of the known fexofenadine base polymorphic forms or a salt thereof. Said pharmaceutical composition can optionally contain a therapeutically effective amount of pseudoephedrine.

The following examples illustrate the invention.

### EXAMPLE 1

### Preparation of fexofenadine free base Form α

A 100 ml round-bottom flask, equipped with magnetic stirrer, is loaded with 10 g (18.6 mmoles) of fexofenadine hydrochloride and 50 ml of methanol, to obtain a clear solution. Afterwards, 2.5 ml of triethylamine (1.81 g; 17.9 mmoles) are added. After about 2 minutes, a precipitate forms which is filtered off, washed with methanol and dried at room temperature, thereby affording 8.9 g (17.7 mmoles) of fexofenadine free base. XRPD analysis of the product shows that it is a novel crystalline form of fexofenadine free base, substantially characterised by the peaks as reported in Figure 1.

### EXAMPLE 2

### Preparation of fexofenadine free base Form ß

A 100 ml round-bottom flask, equipped with magnetic stirrer, is loaded with 10 g (18.6 mmoles) of fexofenadine hydrochloride and 50 ml of methanol, to obtain a clear solution. Afterwards, 5 ml of triethylamine (3.63 g; 35.9 mmoles) are added thereto, then acetic acid is added to acidify again the mixture. After about 2 minutes, a precipitate forms which is filtered off, washed with methanol and dried under vacuum at room temperature, thereby affording 7.9 g (15.8 mmoles) of fexofenadine free base. XRPD analysis of the product shows that it is a novel crystalline form of fexofenadine free base, substantially characterised by the peaks as reported in Figure 2.

### EXAMPLE 3

### Preparation of fexofenadine free base Form γ

A 100 ml round-bottom flask, equipped with magnetic stirrer, reflux condenser and thermometer, is loaded with 10 g of fexofenadine hydrochloride (18.6 mmoles), 30 ml of acetone and 20 ml of acetonitrile. The mixture is warmed to a temperature of 60°C under stirring, to obtain a suspension. Afterwards, 2.5 ml of triethylamine (1.81 g; 17.9 mmoles) are added thereto. From the resulting clear solution, a precipitate immediately forms which is filtered off and repeatedly washed first with the same acetone-acetonitrile mixture as used in the preparation, then with only acetone. Finally the solid product is dried in a static dryer under vacuum at a temperature of 50°C, thereby affording 8.1 g (16.2 mmoles) of fexofenadine free base. XRPD analysis of the product shows that it is a novel crystalline form of fexofenadine free base, substantially characterised by the peaks as reported in Figure 3.

### EXAMPLE 4

### Preparation of Fexofenadine free base form γ

A 50 ml round-bottom flask, equipped with magnetic stirrer, reflux condenser and thermometer, is loaded with 5 g of fexofenadine hydrochloride (9.32 mmoles) and 25 ml of acetone. The mixture is refluxed under stirring, to obtain a suspension. Afterwards, 0.9 ml of butylamine (0.68 g; 9.32 mmoles) are added thereto. From the resulting clear solution, a precipitate immediately forms which is filtered off and repeatedly washed with acetone. Finally the solid product is dried in a static dryer under vacuum at a temperature of 50°C, thereby affording 4.1 g (8.21 mmoles) of Fexofenadine free base. XRPD analysis of the product shows that it is a novel crystalline form of fexofenadine free base, substantially characterised by the peaks as reported in Figure 3.

### EXAMPLE 5

### Preparation of Fexofenadine free base Form γ

A 50 ml round-bottom flask, equipped with magnetic stirrer, reflux condenser and thermometer, is loaded with 5 g of fexofenadine hydrochloride (9.32 mmoles) and 25 ml of acetone. The mixture is refluxed under stirring, to obtain a suspension. Afterwards, 2.22 ml of tributylamine (1.73 g; 9.32 mmoles) are added thereto. From the resulting clear solution, a precipitate immediately forms which is filtered off and repeatedly washed with acetone. Finally the solid product is dried in a static dryer under vacuum at a temperature of 50°C, thereby affording 3.5 g (7 mmoles) of fexofenadine free base. XRPD analysis of the product shows that it is a novel crystalline form of fexofenadine free base, substantially characterised by the peaks as reported in Figure one spectrum with peaks characteristics as substantially reported in figure 3.

## Claims

1. Fexofenadine base crystalline Form γ, having an XRPD spectrum wherein the most intense diffraction peaks fall at 5.63; 12.11; 15.83; 16.91; 20.39 and 24.44 in 2θ.

2. The crystalline form as claimed in claim 1, having the XRPD spectrum substantially as reported in Figure 3.

3. A process for the preparation of fexofenadine base, as claimed in claim 1, comprising the following steps:
- reaction of a fexofenadine addition salt with a basic organic agent in an organic aprotic solvent; and
- separation of the precipitate.

4. A process as claimed in claim 3, wherein the organic aprotic solvent is selected from acetone, diethyl ketone, methyl ethyl ketone, ethyl acetate, butyl acetate, acetonitrile or mixtures thereof; and the basic organic agent is ammonia; an amine of formula N(R₁R₂R₃), wherein at least one of R₁, R₂ and R₃ is a C₁-C₆ alkyl or aryl group, and the others are independently hydrogen or a C₁-C₆ alkyl or aryl group; or a saturated or unsaturated 5- or 6- membered heterocyclic compound, containing at least one nitrogen atom.

5. Fexofenadine base crystalline Form β, having an XRPD spectrum wherein the most intense diffraction peaks fall at 8.28; 10.68; 16.23; 16.80; 19.35 and 21.12 in 2θ.

6. The crystalline form as claimed in claim 5, having the XRPD spectrum substantially as reported in Figure 2.

7. A process for the preparation of fexofenadine base, as claimed in claim 5, comprising the following steps:
- reaction of a fexofenadine addition salt with a basic organic agent in an organic protic solvent;
- acidification of the solution with a carboxylic organic acid to precipitate fexofenadine base Form β; and
- separation of the precipitate.

8. A process as claimed in claim 7, wherein the basic organic agent is ammonia; an amine of formula N(R₁R₂R₃), wherein at least one of R₁, R₂ and R₃ is a C₁-C₆ alkyl or aryl group, and the others are independently hydrogen or a C₁-C₆ alkyl or aryl group; or a saturated or unsaturated 5- or 6- membered heterocyclic compound, containing at least one nitrogen atom; the organic protic solvent is an alcohol; and the organic carboxylic acid is a mono-, bi- or tri- carboxylic acid.

9. Fexofenadine base crystalline Form α, having an XRPD spectrum wherein the most intense diffraction peaks fall at 4.37; 8.75; 10.07; 13.16; 15.65; 16. 22 and 23.57 in 2θ.

10. The crystalline form as claimed in claim 9, having the XRPD spectrum substantially as reported in Figure 1.

11. A process for the preparation of fexofenadine base, as claimed in claim 9, comprising the following steps:
- reaction of a fexofenadine addition salt with a basic organic agent in an organic protic solvent; and
- separation of the precipitate.

12. A process as claimed in claim 11, wherein the basic organic agent is ammonia; an amine of formula N(R₁R₂R₃), wherein at least one of R₁, R₂ and R₃ is a C₁-C₆ alkyl or aryl group, and the others are independently hydrogen or a C₁-C₆ alkyl or aryl group; or a saturated or unsaturated 5- or 6- membered heterocyclic compound, containing at least one nitrogen atom; and the organic protic solvent is an alcohol.

13. The use of fexofenadine free base Form γ, Form β or Form α, as claimed in claim 1, 5 and 9, in a process for the preparation of fexofenadine free base, or a salt or polymorphic form thereof, having a purity degree suitable for the pharmaceutical or veterinary use.

14. A pharmaceutical composition comprising a suitable carrier and/or excipient and, as the active ingredient, at least one of fexofenadine base Form γ, Form β or Form α, as defined in claims 1, 5 and 9, or mixtures thereof or mixtures of at least one of them with one or more of the known polymorphic forms of fexofenadine base or a salt thereof, optionally also containing a therapeutically effective amount of pseudoephedrine.
